(19) Europäisches Patentamt
European Patent Office
Office européen des brevets

(11) EP 2 569 272 B1

(12) **FASCICULE DE BREVET EUROPEEN**

(45) Date de publication et mention de la délivrance du brevet:
**24.08.2016 Bulletin 2016/34**

(21) Numéro de dépôt: **11720429.7**

(22) Date de dépôt: **09.05.2011**

(51) Int Cl.:
***C07C 51/31*** *(2006.01)* ***C07C 51/14*** *(2006.01)*
***C07C 51/02*** *(2006.01)*

(86) Numéro de dépôt international:
**PCT/EP2011/057381**

(87) Numéro de publication internationale:
**WO 2011/141404 (17.11.2011 Gazette 2011/46)**

(54) **PROCEDE DE PREPARATION DE DIACIDES CARBOXYLIQUES**

VERFAHREN ZUR HERSTELLUNG VON DICARBONSÄUREN

PROCESS FOR PREPARING DICARBOXYLIC ACIDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.05.2010 FR 1053628**

(43) Date de publication de la demande:
**20.03.2013 Bulletin 2013/12**

(73) Titulaire: **Rhodia Operations**
**93306 Aubervilliers (FR)**

(72) Inventeurs:
- **CHOUZIER, Sandra**
  **F-69006 Lyon (FR)**
- **MIGNANI, Gérard**
  **F-69008 Lyon (FR)**
- **ROUSSEAU, Simon**
  **F-33170 Gradignan (FR)**
- **SARRAZIN, Flavie**
  **F-33400 Talence (FR)**
- **MASTROIANNI, Sergio**
  **F-69006 Lyon (FR)**

(74) Mandataire: **Chatelan, Florence Anne et al**
**Rhodia Opérations**
**85, avenue des Frères Perret - BP 62**
**69192 Saint-Fons Cedex (FR)**

(56) Documents cités:
**DD-A- 68 897**
**DE-C- 767 840**
**FR-A- 1 346 615**
**GB-A- 1 296 447**
**US-A- 3 076 026**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation de diacides carboxyliques, en particulier d'acide adipique (acide 1,6-hexanedioïque) par action de l'acide nitrique à partir des alcools ou cétones cycliques correspondants du point de vue du nombre d'atomes de carbone, en présence d'un ou plusieurs oxydes d'azote en une concentration molaire importante dans le milieu réactionnel.

L'acide adipique est un produit intermédiaire important, notamment dans le domaine des polymères et plus particulièrement pour la préparation du polyamide, par exemple du polyamide 6-6, ou des polyuréthanes. Pour les applications du polyamide 6-6, il est nécessaire d'avoir une très grande pureté et cette pureté doit déjà exister au stade des précurseurs, notamment au stade de l'acide adipique. L'exigence de pureté maximale est particulièrement accrue lorsque l'acide adipique doit servir de matière première pour les industries textile, électronique ou agro-alimentaire.

Ainsi, que ce soit pour la production de polyamide 6-6 ou pour d'autres applications telles que la production de certains polyuréthannes, la pureté de l'acide adipique mis en oeuvre doit être extrêmement élevée, tant pour les teneurs en sous-produits organiques que pour les teneurs en résidus métalliques. En effet, l'acide adipique ne doit pas contenir d'impuretés, en particulier métallique, en concentration supérieure à 1 ppm.

Castellan et al.(Catalysis Today, 9 (1991) 237-322) propose une large revue des enjeux liés à l'acide adipique, en particulier ceux liés à sa préparation par action d'acide nitrique en présence de catalyseurs d'oxydation comme le vanadium et le cuivre.
En effet, l'acide adipique est classiquement synthétisé par oxydation par l'acide nitrique d'un mélange de cyclohexanone et de cyclohexanol en présence de catalyseurs d'oxydation comme le cuivre et le vanadium.
La mise en oeuvre de tels catalyseurs présente des inconvénients majeurs liés à leur coût d'utilisation, à la nécessité de les recycler lors des étapes ultérieures, à la nécessité de purifier les produits formés ou bien la nécessité de mise en oeuvre d'étapes supplémentaires de lavage et de cristallisation.
Quoi qu'il en soit, en l'absence de tels catalyseurs, la réaction directe de l'acide nitrique conduit principalement à la formation de dérivés azotés stables ($N_2$, $N_2O$), qui sont donc difficilement re-oxydables, ainsi qu'à une médiocre sélectivité en acide adipique du fait de la formation d'autres diacides tels les diacides glutarique et succinique, comme décrit par Castellan et al. *(supra)*.
Ainsi, les procédés connus présentent des inconvénients majeurs parmi lesquels on peut tout particulièrement citer la mise en oeuvre de catalyseurs.

En outre, l'oxydation nitrique par l'acide nitrique ou par des oxydes d'azotes de composés tels que le cyclohexanol génère des vapeurs nitreuses. Ces vapeurs sont générées dans une quantité qui est difficilement contrôlable et peu exploitable puisqu'elle dépend des conditions de mise en oeuvre de la réaction (température, nature et pureté du substrat à oxyder...). Il est ainsi connu d'éliminer ces vapeurs au fur et à mesure de leur formation, comme divulgué dans le document DE-767840.

La présente invention se propose ainsi de fournir un procédé amélioré de mise en oeuvre de la réaction d'oxydation nitrique pour produire des diacides carboxyliques à partir des alcools ou cétones cycliques ayant le même nombre d'atomes de carbone, en présence d'une quantité significative d'oxydes d'azote, et qui permette de résoudre en tout ou partie les problèmes des procédés de l'état de la technique antérieur.
En effet, le procédé selon l'invention est un procédé, qui permet de s'affranchir de la présence de catalyseur, et ainsi diminuer les coûts associés à la purification du diacide carboxylique obtenu, notamment vis à vis de la teneur en métaux résiduels. Il permet également d'éviter les coûts liés aux opérations de recyclage des catalyseurs. Ainsi, du fait de l'absence de catalyseur, le procédé selon l'invention est également un procédé plus respectueux de l'environnement.
Le procédé selon l'invention permet en outre de consommer ou recycler les oxydes d'azote formés, de conserver une sélectivité en acide adipique particulièrement élevée, qui est souvent de l'ordre ou supérieure à 90%, notamment supérieure à 95%, d'obtenir des rendements élevés en diacides qui peuvent atteindre ou dépasser 90%, notamment 95% après 5 minutes, et de permettre de faibles temps de passage des réactifs. Cette sélectivité est obtenue quelque soit le composé de départ à oxyder. En effet, le procédé selon l'invention permet d'atteindre les valeurs susmentionnées de sélectivité en acide adipique à partir de cyclohexanol, de cyclohexanone ou du mélange des deux composés.
Le procédé de l'invention permet également une mise en oeuvre à des températures plus faibles que les procédés connus.
Le procédé selon l'invention permet également de lancer ou de conduire la réaction en présence de la seule cyclohexanone lors de la préparation de l'acide adipique.
Le procédé selon l'invention peut également être un procédé continu ou discontinu de préparation de diacides carboxyliques.

Un objet de la présente invention est donc de fournir un procédé de préparation d'un diacide carboxylique de formule (I),

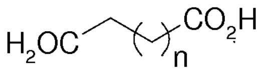

(I)

dans laquelle n représente un entier allant de 1 à 9, par action d'acide nitrique, en présence d'un ou plusieurs oxydes d'azote choisis parmi NO, $NO_2$, $N_2O_3$, $N_2O_4$, $N_2O_5$, $NO_3$, $N_2O_6$, $N_4O$ et leurs mélanges en une concentration molaire dans le milieu réactionnel supérieure à 2,5 mmol par kg de milieu réactionnel, sur les composés de formule (II) ou (III) dans lesquelles n représente le même nombre entier que dans la formule (I)

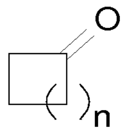

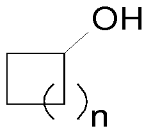

(II) (III)

le ratio molaire R correspondant au nombre de moles d'oxydes d'azote ajoutés au milieu réactionnel par rapport au nombre de moles de substrat à oxyder (composés (II) et/ou (III)) étant supérieur ou égal à 1,5.

Selon un autre aspect de l'invention, le procédé met en oeuvre un mélange des composés de formule (II) et (III).

Selon un autre aspect de l'invention, le procédé selon l'invention est mis en oeuvre pour la préparation d'un composé de formule (I) pour laquelle n représente 2, 3, 4, 5 ou 9, de préférence 3 ou 9, en particulier n représente 3.

Le procédé selon l'invention met en oeuvre de l'acide nitrique, le plus souvent en solution aqueuse, en des quantités de l'ordre de 30 à 70% massique, de préférence de l'ordre de 50 à 60% massique dans le milieu réactionnel, de préférence de l'ordre de 50% massique dans le milieu réactionnel.

Par « en présence d'un ou plusieurs oxydes d'azote en une concentration molaire dans le milieu réactionnel supérieure à » on entend au sens de la présente invention, qu'un ou plusieurs oxydes d'azotes sont ajoutés au milieu réactionnel en une quantité correspondante à cette concentration molaire. Cette concentration molaire est indépendante de la quantité de vapeurs nitreuses qui pourraient se former au cours de l'oxydation nitrique des composés de formule (II) et/ou (III) en présence d'acide nitrique.

Selon un autre aspect de l'invention, le procédé met en oeuvre un ou plusieurs oxydes d'azote choisis de préférence parmi NO et $NO_2$.

Selon un aspect préféré de l'invention, le procédé met en oeuvre un ou plusieurs oxydes d'azote en une quantité supérieure à 30 mmol d'oxydes d'azote par kg de milieu réactionnel.

Selon un autre aspect de l'invention, le procédé met en oeuvre un ou plusieurs oxydes d'azote en une quantité allant de 2,5 à 1 000 mmol d'oxydes d'azote par kg de milieu réactionnel, notamment allant de 30 à 1000 mmol d'oxydes d'azote par kg de milieu réactionnel, de manière préférée allant de 30 à 850 mmol d'oxydes d'azote par kg de milieu réactionnel, de manière plus préférée allant de 85 à 700 mmol d'oxydes d'azote par kg de milieu réactionnel, de manière encore plus préférée allant de 180 à 650 mmol d'oxydes d'azote par kg de milieu réactionnel. De façon particulièrement avantageuse, la quantité d'oxydes d'azote ajoutée va de 300 à 400 mmol/kg de milieu réactionnel.

Selon un mode particulièrement avantageux de l'invention, le procédé met en oeuvre un ratio molaire R supérieur ou égal à 2. A partir d'un ratio molaire supérieur ou égal à 0,5, la sélectivité en diacide carboxylique est améliorée. Cet effet est d'autant plus important dès lors que le ratio R est supérieur ou égal à 1,5. La valeur limite supérieure est, pour des raisons économiques et de mise en oeuvre, inférieure ou égale à 50, de préférence inférieure ou égale à 30, notamment inférieure ou égale à 10.

Selon un autre aspect de l'invention, le procédé met en oeuvre un ou plusieurs oxydes d'azote sous forme gazeuse.

Selon un autre aspect de l'invention, le procédé met en oeuvre $NaNO_2$ comme source d'oxyde d'azote.

Le procédé de l'invention met en oeuvre les composés de formule (II) et/ou (III) dans une réaction en une seule étape conduisant directement et majoritairement au diacide carboxylique de formule (I). Ce procédé présente l'avantage de ne pas nécessiter de séparation d'intermédiaires de synthèse, contrairement au procédé décrit dans US 3076026.

Comme indiqué précédemment, le procédé selon l'invention est mis en oeuvre de préférence en l'absence de catalyseur métallique d'oxydation. Ce mode de réalisation constitue un mode très avantageux puisque tout en permettant d'atteindre une sélectivité en acide adipique très satisfaisante, cette absence permet de diminuer les coûts associés à la purification du diacide carboxylique obtenu, notamment vis à vis de la teneur en métaux résiduels. Il permet également d'éviter les

coûts liés aux opérations de recyclage des catalyseurs. Ainsi, du fait de l'absence de catalyseur, le procédé selon l'invention est également un procédé plus respectueux de l'environnement.

Le procédé selon l'invention peut également mettre en oeuvre un catalyseur d'oxydation sans que les avantages du procédé ne soient remis en cause. La présence éventuelle d'un tel catalyseur peut encore améliorer l'efficacité du procédé selon l'invention.

Ainsi selon un autre aspect de l'invention, le procédé met également en oeuvre un catalyseur d'oxydation qui peut être choisi parmi les catalyseurs métalliques. Le catalyseur peut avantageusement être choisi parmi ceux comprenant un élément du groupe consistant en Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et leurs combinaisons.

Lorsqu'ils sont mis en oeuvre, ces éléments catalytiques sont mis en oeuvre soit sous forme de composés avantageusement au moins partiellement solubles dans le milieu liquide d'oxydation aux conditions de mise en oeuvre de la réaction d'oxydation, soit supportés, absorbés ou liés à un support inerte tel que silice, alumine, par exemple.

Lorsqu'il est utilisé, le catalyseur est de préférence soluble dans l'un de ces milieux à température ambiante ou à la température de recyclage de ces milieux dans une nouvelle oxydation. Par le terme soluble, on entend que le catalyseur soit au moins partiellement soluble dans le milieu considéré. Dans le cas d'une catalyse hétérogène, les éléments métalliques catalytiquement actifs sont supportés ou incorporés dans une matrice minérale micro ou mesoporeuse ou dans une matrice polymérique ou sont sous forme de complexes organométalliques liés à un support organique ou minéral. Par « incorporé », on entend que le métal est un élément du support ou que l'on travaille avec des complexes stériquement piégés dans des structures poreuses dans les conditions de l'oxydation. Selon un autre aspect de l'invention, le catalyseur peut être présent en des concentrations en métal dans le milieu liquide d'oxydation allant de 0,00001 à 5% en poids, par exemple allant de 0,001 à 2 % en poids.

Selon un autre aspect de l'invention, le procédé est mis en oeuvre à une température allant de 20 à 150°C, de manière préférée allant de 30 à 100°C, de manière plus préférée allant de 50 à 90°C, par exemple à 50°C ou à 70°C.

Selon un autre aspect de l'invention, le procédé est mis en oeuvre à pression atmosphérique. Toutefois, il est généralement mis en oeuvre sous pression pour maintenir les composants du milieu réactionnel sous forme liquide. Ainsi, la pression peut aller de 1 à 200 bar, de manière préférée de 1 à 100 bar.

Selon un autre aspect de l'invention, le procédé est mis en oeuvre à une pression totale dans le milieu allant de 1 à 30 bar, de manière préférée allant de 1 à 15 bar, de manière plus préférée allant de 1 à 4 bar.

De manière habituelle, le procédé selon l'invention est conduit en milieu aqueux.

Selon un autre aspect de l'invention, les diacides carboxyliques formés, notamment l'acide adipique, sont récupérés à partir de la phase aqueuse, par exemple, par cristallisation. Les diacides carboxyliques ainsi récupérés sont avantageusement purifiés selon les techniques habituelles et décrites dans de nombreux documents. Parmi les procédés de purification, la purification par cristallisation dans différents solvants tel que l'eau, les solutions aqueuses d'acide acétique ou les alcools, est préférée.

De manière avantageuse, le procédé selon l'invention peut être mis en oeuvre au sein d'un procédé plus global pour la préparation d'un diacide carboxylique à partir d'un hydrocarbure, en particulier un hydrocarbure cycloaliphatique ou arylaliphatique, par exemple le cyclohexane ou le cyclododécane. L'hydrocarbure est alors oxydé pour former un alcool ou une cétone qui sont ensuite oxydés pour former l'acide dicarboxylique ou diacide carboxylique selon l'invention. Ainsi, le procédé selon l'invention permet la préparation de diacides carboxyliques linéaires, par exemple de l'acide adipique ou de l'acide dodécanoïque à partir du cyclohexane ou du cyclododécane.

Les exemples suivants illustrent le procédé de l'invention, en particulier les avantages de ce procédé.

## Exemples

### 1. Mode opératoire

- Une solution d'acide nitrique à 68% est préparée avec 350 ppm de vanadium et 5400 ppm de cuivre, une autre solution à 68% est préparée sans cuivre ni vanadium.
- Instant to : on introduit 2,6 mL d'une des deux solutions d'acide nitrique dans un vial de 20 mL. Le vial est ensuite fermé avec un bouchon muni d'un septum, mis sous agitation et chauffé à une température T (cf. valeurs dans le tableau 1).
- Instant to + 30 minutes : on injecte à travers le septum 0,28 mL d'une solution de nitrite de sodium ($NaNO_2$) permettant de générer une quantité molaire d'oxydes d'azote dans le milieu réactionnel à la concentration $C(NO_x)$ en mmol par kg (cf. valeurs dans le tableau 1).
- Instant to + 60 minutes : on injecte à travers le septum 0,5 mL de solution de cyclohexanone à 8% dans l'eau (notée One), ou une solution de cyclohexanol à 4% dans l'eau (notée OI), ou un mélange cyclohexanol/cyclohaxanone

2%/2,2% (noté Ol/One).

- Instant to + 60 minutes + temps de réaction tr : on prélève à travers le septum un échantillon du milieu réactionnel qui subit une trempe à l'eau froide et est analysé par chromatographie HPLC pour quantifier les quantités de diacides produits et de substrat (cyclohexanone et/ ou cyclohexanol) consommé.

2. Définition des grandeurs calculées

- TT : taux de transformation de la cyclohexanone (% molaire) :

$$TT = \frac{Ncyclohexanone(initial) - Ncyclohexanone(tr)}{Ncyclohexanone(initial)} \times 100$$

- RT : rendement en acide adipique (% molaire)

$$RT = \frac{Nacide\ adipique(tr)}{Ncyclohexanone(initial)} \times 100$$

- Sélectivité : proportion d'acide adipique formé par rapport aux trois diacides dosés : acides succinique, glutarique et adipique (% molaire) :

$$Sélectivité = \frac{Nacide\ adipique(tr)}{Nacide\ succinique(tr) + Nacide\ glutarique(tr) + Nacide\ adipique(tr)} \times 100$$

- R : Ratio du nombre de moles d'oxydes d'azote ajoutés au milieu réactionnel par rapport au nombre de moles de substrat à oxyder :

$$R = \frac{NNOx(initial)}{Ncyclohexanone(initial)}$$

Nx(t) représente le nombre de moles du composé x dans le milieu réactionnel à l'instant t.

Remarque : les TT, RT et R des autres types de substrat, à savoir le cyclohexanol ou le mélange cyclohexanol/cyclohexanone sont calculés de la même manière que ci-dessus, le Ncyclohexanone étant remplacé par Nsubstrat.

3. Résultats

Les résultats en présence et absence de catalyseurs, pour différentes températures T, concentrations en oxydes d'azote ajoutés $C(NO_x)$, nature de substrat et temps de réaction tr sont consignés dans les tableaux 1 à 7 suivants :

Influence de la présence de catalyseur :

Tableau 1

| N° | Présence de Cu et V | Substrat injecté | $C(NO_x)$ (mmol/kg) | R | T (°C) | tr (min) | TT (%molaire) | RT (%molaire) | Sélectivité (%molaire) |
|---|---|---|---|---|---|---|---|---|---|
| 1 | oui | One | 0 | 0 | 70 | 60 | 100 | 26 | 69 |
| 2 | oui | One | 350 | 3,89 | 50 | 4 | 100 | 81 | 98 |
| 3 | non | One | 182 | 1,95 | 70 | 60 | 100 | 77 | 92 |
| 4 | non | One | 350 | 3,89 | 50 | 5 | 100 | 89 | 97 |

(suite)

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 5 | non | One | 650 | 7,79 | 70 | 60 | 100 | 85 | 93 |

En comparant les essais 2 et 4, on constate que la présence de catalyseur n'est pas nécessaire afin d'atteindre une sélectivité en acide adipique très satisfaisante (supérieure à 90 %).

Influence de la nature du substrat :

Tableau 2

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 4 | non | One | 350 | 3,89 | 50 | 5 | 100 | 89 | 97 |
| 6 | non | Ol/One | 350 | 7,34 | 70 | 60 | 100 | 95 | 95 |
| 7 | non | Ol | 350 | 7,94 | 70 | 10 | 100 | 95 | 96 |

On constate quelle que soit la nature du substrat, on obtient une sélectivité supérieure ou égale à 95%.

Influence de la température :

Tableau 3

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 4 | non | One | 350 | 3,89 | 50 | 5 | 100 | 89 | 97 |
| 8 | non | One | 350 | 3,89 | 20 | 60 | 100 | 82 | 95 |
| 7 | non | Ol | 350 | 7,94 | 70 | 10 | 100 | 95 | 96 |
| 9 | non | Ol | 350 | 7,94 | 20 | 60 | 100 | 78 | 93 |

On constate que le procédé de l'invention permet d'atteindre une sélectivité supérieure à 90 % même à température ambiante. La réaction est plus rapide lorsque la température est supérieure ou égale à 50°C

Influence de la quantité de NOx ajoutée :

Avec catalyseur : En comparant les essais 1 et 2 du tableau 1 ci-avant, on constate que, en présence de catalyseur, la sélectivité augmente d'autant plus que la quantité de NOx ajoutée est élevée.

Sans catalyseur /One :

Tableau 4

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 3 | non | One | 182 | 1,95 | 70 | 60 | 100 | 77 | 92 |
| 4 | non | One | 350 | 3,89 | 50 | 5 | 100 | 89 | 97 |

(suite)

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 5 | non | One | 650 | 7,79 | 70 | 60 | 100 | 85 | 93 |

Sans catalyseur /Ol/One :

Tableau 5

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 6 | non | Ol/One | 350 | 7,34 | 70 | 60 | 100 | 95 | 95 |

Sans catalyseur /Ol :

Tableau 6

| N° | Présence de Cu et V | Substrat injecté | C($NO_x$) (mmol/kg) | R | T (°C) | tr (min) | TT (%molaire) | RT (%molaire) | Sélectivité (%molaire) |
|---|---|---|---|---|---|---|---|---|---|
| 10 | non | Ol | 182 | 3,97 | 70 | 60 | 100 | 95 | 95 |
| 7 | non | Ol | 350 | 7,94 | 70 | 10 | 100 | 95 | 96 |
| 11 | non | Ol | 650 | 15,88 | 70 | 60 | 100 | 93 | 95 |

On constate que la sélectivité est améliorée dès le premier ajout de NOx. On constate aussi qu'en absence de catalyseur, la sélectivité augmente d'autant plus que la quantité de NOx ajoutée est élevée. Une sélectivité supérieure à 90 % est atteinte quelque soit le substrat de départ dès lors que l'on ajoute au moins 182 mmol/kg d'oxydes d'azote. Pour le cyclohexanol, une sélectivité supérieure à 90 % est atteinte dès lors que l'on ajoute au moins 30 mmol/kg d'oxydes d'azote.

4. Conclusion

Le procédé selon l'invention permet donc d'atteindre un taux de transformation du substrat organique de 100% tout en permettant des rendements élevés en acide adipique et une sélectivité remarquable, sans qu'il soit nécessaire d'utiliser de catalyseur.

## Revendications

1. Procédé de préparation d'un diacide carboxylique de formule (I),

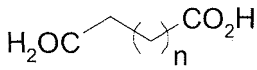

(I)

dans laquelle n représente un entier allant de 1 à 9, en une seule étape, par action d'acide nitrique, en présence d'un ou plusieurs oxydes d'azote choisis parmi NO, $NO_2$, $N_2O_3$, $N_2O_4$, $N_2O_5$, $NO_3$, $N_2O_6$, $N_4O$ et leurs mélanges en une concentration molaire dans le milieu réactionnel supérieure à 2,5 mmol par kg de milieu réactionnel, sur les composes de formule (II) ou (III) dans lesquelles n représente le même nombre entier que dans la formule (I).

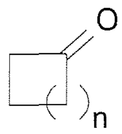
(II)

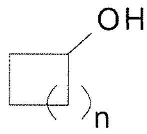
(III)

**caractérisé en ce que** le ratio molaire R correspondant au nombre de moles d'oxydes d'azote ajoutés au milieu réactionnel par rapport au nombre de moles de substrat à oxyder (composés de formule (II) et/ou (III)) est supérieur ou égal à 1,5.

**2.** Procédé selon la revendication 1 mis en oeuvre sur un mélange des composes de formule (II) et (III).

**3.** Procédé selon les revendications 1 ou 2 pour lequel n représente 3 ou 9.

**4.** Procédé selon les revendications 1 à 3 **caractérisé en ce que** le ratio molaire R est supérieur ou égal à 2.

**5.** Procédé selon les revendications 1 à 4 pour lequel le ou les oxydes d'azote sont choisis parmi NO et $NO_2$.

**6.** Procédé selon les revendications 1 à 5 pour lequel la quantité d'oxydes d'azote est supérieure à 30 mmol/kg de milieu réactionnel.

**7.** Procédé selon les revendications 1 à 6 pour lequel la quantité d'oxydes d'azote va de 30 à 1000 mmol/kg de milieu réactionnel.

**8.** Procédé selon les revendications 1 à 7 pour lequel la quantité d'oxydes d'azote va de 30 à 850 mmol/kg de milieu réactionnel.

**9.** Procédé selon les revendications 1 à 8 pour lequel la quantité d'oxydes d'azote va de 180 à 650 mmol/kg de milieu réactionnel.

**10.** Procédé selon les revendications 1 à 9 pour lequel la quantité d'oxydes d'azote va de 300 à 400 mmol/kg de milieu réactionnel.

**11.** Procédé selon les revendications 1 à 10 pour lequel le ou les oxydes d'azote sont mis en oeuvre sous forme gazeuse.

**12.** Procédé selon les revendications 1 à 11 mis en oeuvre en l'absence de catalyseur métallique d'oxydation.

**13.** Procédé selon les revendications 1 à 12 qui met également en oeuvre un ou plusieurs catalyseurs d'oxydation.

**14.** Procédé selon les revendications 1 à 13 mis en oeuvre à une température allant de 20 à 150°C.

**15.** Procédé selon les revendications 1 à 14 mis en oeuvre à ne température allant de 50 à 90°C.

**16.** Procédé selon les revendications 1 à 15 mis en oeuvre à une pression totale dans le milieu allant de 1 à 30 bar.

**17.** Procédé selon les revendications 1 à 16 mis en oeuvre à une pression totale dans le milieu allant de 1 à 4 bar.

**18.** Procédé selon les revendications 1 à 10 et 12 à 17 mettant en oeuvre $NaNO_2$ comme source d'oxyde d'azote.

## Patentansprüche

**1.** Verfahren zur Herstellung einer Dicarbonsäure der Formel (I),

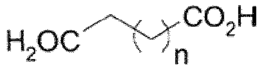

(I)

in der n für eine ganze Zahl im Bereich von 1 bis 9 steht, in einem einzigen Schritt durch Einwirkung von Salpetersäure in Gegenwart eines oder mehrerer Stickstoffoxide, die aus NO, $NO_2$, $N_2O_3$, $N_2O_4$, $N_2O_5$, $NO_3$, $N_2O_6$, $N_4O$ und Mischungen davon ausgewählt sind, in einer molaren Konzentration im Reaktionsmedium von mehr als 2,5 mmol pro kg Reaktionsmedium, auf die Verbindungen der Formel (II) oder (III), in denen n für die gleiche ganze Zahl wie in der Formel (I) steht,

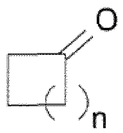

(II)

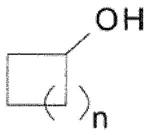

(III)

**dadurch gekennzeichnet, dass** das Molverhältnis R, das der Zahl der Mole von dem Reaktionsmedium zugesetzten Stickstoffoxiden im Verhältnis zur Zahl der Mole von zu oxidierendem Substrat (Verbindungen der Formel (II) und/oder (III)) entspricht, größer gleich 1,5 ist.

2. Verfahren nach Anspruch 1, das an einer Mischung von Verbindungen der Formel (II) und (III) durchgeführt wird.

3. Verfahren nach Anspruch 1 oder 2, für das n für 3 oder 9 steht.

4. Verfahren nach den Ansprüchen 1 bis 3, **dadurch gekennzeichnet, dass** das Molverhältnis R größer gleich 2 ist.

5. Verfahren nach den Ansprüchen 1 bis 4, für das das bzw. die Stickstoffoxide aus NO und $NO_2$ ausgewählt werden.

6. Verfahren nach den Ansprüchen 1 bis 5, für das die Menge von Stickstoffoxiden größer als 30 mmol/kg Reaktionsmedium ist.

7. Verfahren nach den Ansprüchen 1 bis 6, für das die Menge von Stickstoffoxiden 30 bis 1000 mmol/kg Reaktionsmedium beträgt.

8. Verfahren nach den Ansprüchen 1 bis 7, für das die Menge von Stickstoffoxiden 30 bis 850 mmol/kg Reaktionsmedium beträgt.

9. Verfahren nach den Ansprüchen 1 bis 8, für das die Menge von Stickstoffoxiden 180 bis 650 mmol/kg Reaktionsmedium beträgt.

10. Verfahren nach den Ansprüchen 1 bis 9, für das die Menge von Stickstoffoxiden 300 bis 400 mmol/kg Reaktionsmedium beträgt.

11. Verfahren nach den Ansprüchen 1 bis 10, für das das bzw. die Stickstoffoxide in Gasform eingesetzt werden.

12. Verfahren nach den Ansprüchen 1 bis 11, das in Abwesenheit von metallischem Oxidationskatalysator durchgeführt wird.

13. Verfahren nach den Ansprüchen 1 bis 12, bei dem außerdem ein oder mehrere Oxidationskatalysatoren verwendet werden.

14. Verfahren nach den Ansprüchen 1 bis 13, das bei einer Temperatur im Bereich von 20 bis 150°C durchgeführt wird.

15. Verfahren nach den Ansprüchen 1 bis 14, das bei einer Temperatur im Bereich von 50 bis 90°C durchgeführt wird.

16. Verfahren nach den Ansprüchen 1 bis 15, das bei einem Gesamtdruck im Medium im Bereich von 1 bis 30 bar durchgeführt wird.

17. Verfahren nach den Ansprüchen 1 bis 16, das bei einem Gesamtdruck im Medium im Bereich von 1 bis 4 bar durchgeführt wird.

18. Verfahren nach den Ansprüchen 1 bis 10 und 12 bis 17, bei dem $NaNO_2$ als Stickstoffoxid-Quelle verwendet wird.

## Claims

1. Process for preparing a dicarboxylic acid of formula (I),

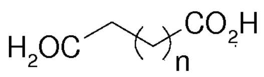

(I)

in which n represents an integer from 1 to 9, in a single step, by action of nitric acid, in the presence of one or more oxides of nitrogen selected from NO, $NO_2$, $N_2O_3$, $N_2O_4$, $N_2O_5$, $NO_3$, $N_2O_6$, $N_4O$ and mixtures thereof at a molar concentration in the reaction mixture of greater than 2.5 mmol per kg of reaction mixture, on the compounds of formula (II) or (III) in which n represents the same integral number as in the formula (I)

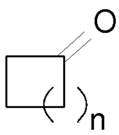 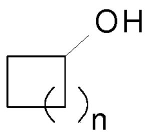

(II) (III)

**characterized in that** the molar ratio R, corresponding to the number of moles of oxides of nitrogen added to the reaction mixture relative to the number of moles of substrate to be oxidized (compounds (II) and/or (III)), is greater than or equal to 1.5.

2. Process according to Claim 1, employed on a mixture of compounds of formula (II) and (III).

3. Process according to Claims 1 or 2, for which n represents 3 or 9.

4. Process according to Claims 1 to 3, **characterized in that** the molar ratio R is greater than or equal to 2.

5. Process according to Claims 1 to 4, for which the oxide or oxides of nitrogen are selected from NO and $NO_2$.

6. Process according to Claims 1 to 5, for which the amount of oxides of nitrogen is greater than 30 mmol/kg of reaction mixture.

7. Process according to Claims 1 to 6, for which the amount of oxides of nitrogen is from 30 to 1000 mmol/kg of reaction mixture.

8. Process according to Claims 1 to 7, for which the amount of oxides of nitrogen is from 30 to 850 mmol/kg of reaction mixture.

9. Process according to Claims 1 to 8, for which the amount of oxides of nitrogen is from 180 to 650 mmol/kg of reaction mixture.

**10.** Process according to Claims 1 to 9, for which the amount of oxides of nitrogen is from 300 to 400 mmol/kg of reaction mixture.

**11.** Process according to Claims 1 to 10, for which the oxide or oxides of nitrogen are employed in gaseous form.

**12.** Process according to Claims 1 to 11, employed in the absence of metallic oxidation catalyst.

**13.** Process according to Claims 1 to 12, further employing one or more oxidation catalysts.

**14.** Process according to Claims 1 to 13, employed at a temperature of from 20 to 150°C.

**15.** Process according to Claims 1 to 14, employed at a temperature of from 50 to 90°C.

**16.** Process according to Claims 1 to 15, employed at a total pressure in the mixture of from 1 to 30 bar.

**17.** Process according to Claims 1 to 16, employed at a total pressure in the mixture of from 1 to 4 bar.

**18.** Process according to Claims 1 to 10 and 12 to 17, employing $NaNO_2$ as source of oxide of nitrogen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 767840
- US 3076026 A


### Littérature non-brevet citée dans la description


- **CASTELLAN et al.** *Catalysis Today,* 1991, vol. 9, 237-322